# EUROPEAN PATENT SPECIFICATION

(11) **EP 3 531 907 B1**
(45) Date of publication and mention of the grant of the patent: **29.11.2023**
(21) Application number: 18797602.2
(22) Date of filing: 08.05.2018
(51) Int. Cl.: A61B 5/022, A61B 5/00, H01R 13/62, H01R 24/38, H01R 35/04, H01R 43/26

(54) **FINGER CUFF CONNECTOR**
FINGERMANSCHETTENVERBINDER
DISPOSITIF DE RACCORDEMENT DE MANCHON DE DOIGT

(30) Priority: 09.05.2017 US 201762503610 P; 18.04.2018 US 201815955939
(43) Date of publication of application: 04.09.2019
(73) Proprietor: Edwards Lifesciences Corporation, Irvine, CA 92614 (US)
(72) Inventor: AXELROD, Blake W., Irvine, CA 92614 (US); SIEMONS, Alexander H., Irvine, CA 92614 (US)
(74) Representative: Eisenführ Speiser
(86) International application number: PCT/US2018/031491
(87) International publication number: WO 2018/208713

(56) References cited:
- WO-A1-2012/160477
- JP-A- H08 332 172
- KR-B1- 100 659 162
- US-A1- 2006 058 687
- US-A1- 2007 079 982
- US-A1- 2010 016 737
- US-A1- 2011 046 494
- US-A1- 2011 105 917
- US-A1- 2014 371 607

## Description

### BACKGROUND

### Field

Embodiments of the invention relate to a finger cuff connector for a blood pressure measurement system that includes a finger cuff that utilizes volume clamping.

### Relevant Background

Volume clamping is a technique for non-invasively measuring blood pressure in which pressure is applied to a subject's finger in such a manner that venous flow is fully obstructed and arterial pressure may be balanced by a time varying pressure to maintain a constant arterial volume. In a properly fitted and calibrated system, the applied time varying pressure is equal to the arterial blood pressure in the finger. The applied time varying pressure may be measured to provide a reading of the patient's arterial blood pressure.

This may be accomplished by a finger cuff that is arranged around a finger of a patient. The finger cuff may include an infrared light source, an infrared sensor, and an inflatable bladder. The infrared light may be sent through the finger in which a finger artery is present. The infrared sensor picks up the infrared light and the amount of infrared light registered by the sensor may be inversely proportional to the artery diameter and indicative of the pressure in the artery.

In the finger cuff implementation, by inflating the bladder in the finger cuff, a pressure is exerted on the finger artery. If the pressure is high enough, it will compress the artery and the amount of light registered by the sensor will increase. The amount of pressure necessary in the inflatable bladder to compress the artery is dependent on the blood pressure. By controlling the pressure of the inflatable bladder such that the diameter of the finger artery is kept constant, the blood pressure may be monitored in very precise detail as the pressure in the inflatable bladder is directly linked to the blood pressure.

In a typical present day finger cuff implementation, a volume clamp system is used with the finger cuff. The volume clamp system typically includes a pressure generating system and a regulating system that includes: a pump, a valve, and a pressure sensor in a closed loop feedback system that are used in the measurement of the arterial volume. To accurately measure blood pressure, the feedback loop provides sufficient pressure generating and releasing capabilities to match the pressure oscillations of the subject's blood pressure.

In present day implementations, the pressure generating and regulating system is located remotely from the clamped finger. A cable containing an air pressure line and electrical connections for the arterial volume measurement connects the pressure generating and regulating system to the finger cuff that applies pressure to the finger. The physical interaction between the finger cuff and the patient's finger is critical for achieving a properly fitted and calibrated system such that the pressure in the finger cuff is equal to the pressure in the patient's artery (e.g., such that the transmural pressure drop is negligible). Mechanical forces exerted on the finger cuff by the cable can affect the fit and interaction between the finger cuff and the patient's finger and thereby interfere with accurate, continuous blood pressure measurement.

US 2011/0046494 A1 discloses a blood pressure cuff equipped with an electronic component providing encoding of cuff properties. The connectors and hoses connecting the cuff to a blood pressure measurement instrument are provided with conductors and electrical coupling, allowing the measuring instrument to access the electronic component encoding cuff properties. The arrangement of the cuff, hose, and connectors makes simultaneous pneumatic and electrical connection when the cuff is attached to the hose.

US 2010/0016737 A1 discloses a blood pressure system that includes a compact monitor housing that contains a pneumatic circuit and an electrical circuit and a processor for inflating and deflating a cuff to provide blood pressure related data. A split connector allows the housing to interface both pneumatically and electronically with one or more independent cuffs and/or with one or more host stations.

WO 2012/160477 A1 discloses a connection assembly for rotatably and electrically coupling an accessory to a main device including a housing portion, a rotatable port assembly, and a wire assembly. An end of the port assembly is coupled to the accessory. The port assembly includes an electrical connector member having a first connector end electrically coupled to the accessory. The wire assembly has an electrical wire member having a first end electrically coupled to a second connector end of the electrical connector member and a second end electrically coupled to a power supply. The wire member is spooled around the port assembly and encased in a chamber defined between the housing portion and the port assembly. Rotation of the port assembly in opposite directions causes the electrical wire member to spool more and less tightly around the port assembly without causing pinching or tangling of wires.

US 2007/0079982 A1 discloses a connector to couple a breathing conduit to a patient interface or another conduit. The connector allows an electrical connection in a first conduit to be connected to another electrical connection in a patient interface or second conduit. The connector is capable of swiveling without disrupting the gas flow through the conduits of the electrical connection.

### SUMMARY

Embodiments of the invention may relate to a connector for a blood pressure measurement system that includes a pressure generating and regulating system and a finger cuff, in which the connector comprises: a first half portion pneumatically and electrically connected to the pressure generating and regulating system; and a second half portion fixedly attached to the finger cuff, wherein the first half portion and the second half portion are connectable in two or more orientations, and wherein the pressure generating and regulating system and the finger cuff are pneumatically and electrically connected when the first half portion and the second half portion are connected, wherein the first half portion and the second half portion comprise one or more mechanical key features that facilitate alignment of the first half portion and the second half portion, wherein the mechanical key features facilitate alignment of the first half portion and the second half portion in two or more discrete possible angular orientations, wherein the connector comprises electrical connector pads being commensurate with the number of possible orientations such that there is a set of electrical connector pads in the second half portion that makes suitable contact with electrical connector pins in the first half portion when the two halves are connected in any of the possible orientations.

### BRIEF DESCRIPTION OF THE DRAWINGS

FIG. 1 is a diagram of a blood pressure measurement device, according to one embodiment of the invention.
FIGs. 2A, 2B, and 2C are diagrams illustrating a finger cuff connector pair according to the invention.
FIGs. 3A and 3B are diagrams illustrating finger cuff connector pairs connected in two different orientations according to the invention.
FIGs. 4A, 4B, 4C, and 4D are diagrams illustrating finger cuff connector pairs connected in four different orientations according to the inventnion.
FIGs. 5A and 5B are diagrams illustrating an example finger cuff connector pair in which the first half portion of the finger cuff connector pair is continuously rotatable relative to the second half portion of the finger cuff connector pair.

### DETAILED DESCRIPTION

Embodiments of the invention may relate to a connector for a blood pressure measurement system that includes a pressure generating and regulating system and a finger cuff, in which the connector comprises: a first half portion pneumatically and electrically connected to the pressure generating and regulating system; and a second half portion fixedly attached to the finger cuff, wherein the first half portion and the second half portion are connectable in two or more orientations, and wherein the pressure generating and regulating system and the finger cuff are pneumatically and electrically connected when the first half portion and the second half portion are connected, wherein the first half portion and the second half portion comprise one or more mechanical key features that facilitate alignment of the first half portion and the second half portion, wherein the mechanical key features facilitate alignment of the first half portion and the second half portion in two or more discrete possible angular orientations, wherein the connector comprises electrical connector pads being commensurate with the number of possible orientations such that there is a set of electrical connector pads in the second half portion that makes suitable contact with electrical connector pins in the first half portion when the two halves are connected in any of the possible orientations.

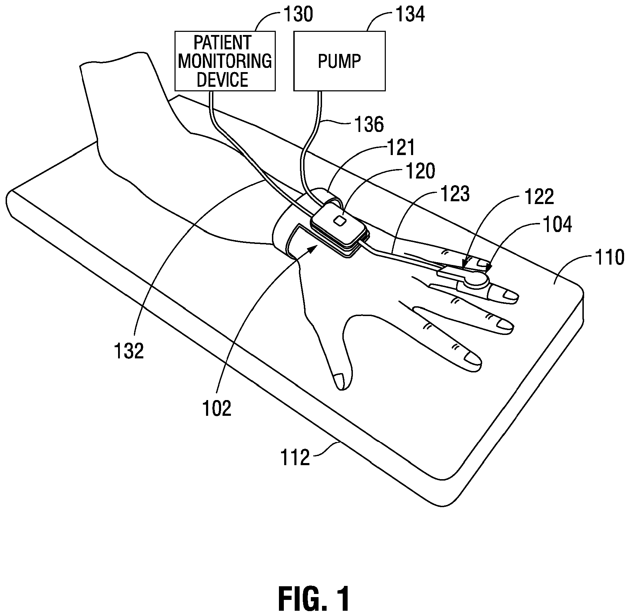

With reference to FIG. 1, an example of a blood pressure measurement device 102 will be described. Finger cuff connectors 122 in accordance with embodiments of the invention may be utilized with the blood pressure measurement device 102. As shown in FIG. 1, the blood pressure measurement device 102 may include a finger cuff 104 having a suitable structure that may be attached to a patient's finger and a blood pressure measurement controller 120 that may be attached to the patient's body (e.g., a patient's hand). The blood pressure measurement device 102 may further be connected to a patient monitoring device 130, and, in some embodiments, a pump 134. Further, finger cuff 104 may include a bladder (not shown) and an LED-PD pair (not shown), which are conventional for finger cuffs.

In one embodiment, blood pressure measurement device 102 may include a pressure measurement controller 120 that includes: a small internal pump, a small internal valve, a pressure sensor, and control circuity. In this embodiment, the control circuitry may be configured to: control the pneumatic pressure applied by the internal pump to the bladder of the finger cuff 104 to replicate the patient's blood pressure based upon measuring the pleth signal received from the LED-PD pair of the finger cuff 104. Further, the control circuitry may be configured to: control the opening of the internal valve to release pneumatic pressure from the bladder; or the internal valve may simply be an orifice that is not controlled. The finger cuff connector 122 passes on the pneumatic pressure received through tube 123 from blood pressure measurement controller 120 to the bladder of finger cuff 104. Additionally, the control circuitry may be configured to: measure the patient's blood pressure by monitoring the pressure of the bladder based upon the input from a pressure senor, which should be the same as patient's blood pressure, and may display the patient's blood pressure on the patient monitoring device 130.

In another embodiment, a conventional pressure generating and regulating system may be utilized, in which, a pump 134 is located remotely from the body of the patient. In this embodiment, the blood pressure measurement controller 120 receives pneumatic pressure from remote pump 134 through tube 136 and passes on the pneumatic pressure through tube 123 and through finger cuff connector 122 to the bladder of finger cuff 104. Blood pressure measurement device controller 120 may also control the pneumatic pressure (e.g., utilizing a controllable valve) applied to the finger cuff 104, as well as other functions. In this example, the pneumatic pressure applied by the pump 134 to the bladder of finger cuff 104 to replicate the patient's blood pressure based upon measuring the pleth signal received from the LED-PD pair of the finger cuff 104 and measuring the patient's blood pressure by monitoring the pressure of the bladder may be controlled by a remote computing device and/or the blood pressure measurement controller 120 and/or the patient monitoring device 130 itself, where the patient monitoring device 130 may also display the patient's blood pressure.

It should be appreciated that embodiments of the invention related to finger cuff connector 122 may be utilized with blood pressure measurement controller 120 having a small internal pump and control circuitry, as previously described, or with conventional pressure generating and regulating systems that include a remote pump 134 and remote processing, or any combinations thereof. Further, it should be appreciated that, in some embodiments, a blood pressure measurement controller 120 is not used at all and there is simply a connection from the tube 123 to finger cuff connector 122 from a remote pump 134 including a remote pressure regulatory system, and all processing for the pressure generating and regulatory system, data processing, and display is performed by a remote computing device. The operations of the blood pressure measurement device 102 including the finger cuff 104 and the blood pressure measurement controller 120 will be hereafter described in more detail with respect to the blood pressure measurement controller 120 having an internal small pump and control circuity, although, it should be appreciated that finger cuff connector 122 may be utilized in a similar manner with a conventional pressure generating and regulating systems that include a remote pump 134 and remote processing.

Continuing with this example, as shown in FIG. 1, a patient's hand may be placed on the face 110 of an arm rest 112 for measuring a patient's blood pressure with the blood pressure measurement device 102. The blood pressure measurement controller 120 of the blood pressure measurement device 102 may be coupled to a bladder of the finger cuff 104 through a finger cuff connector 122 in order to provide pneumatic pressure to the bladder for use in blood pressure measurement. Blood pressure measurement controller 120 may be coupled to the patient monitoring device 130 through a power/data cable 132. Also, in one embodiment, as previously described, in a remote implementation, blood pressure measurement controller 120 may be coupled to a remote pump 134 through tube 136 to receive pneumatic pressure for the bladder of the finger cuff 104. The patient monitoring device 130 may be any type of medical electronic device that may read, collect, process, display, etc., physiological readings/data of a patient including blood pressure, as well as any other suitable physiological patient readings. Accordingly, power/data cable 132 may transmit data to and from patient monitoring device 130 and also may provide power from the patient monitoring device 130 to the blood pressure measurement controller 120 and finger cuff 104.

In one embodiment, a heart reference sensor (HRS) may be placed near the patient's heart level and connected by an HRS connector to the blood pressure measurement controller 120 of the blood pressure measurement device 102 to allow for the compensation of potential errors due to differences in height between the finger cuff 104 and the heart level in the calculation of blood pressure measurements.

As can be seen in FIG. 1, in one example, the finger cuff 104 may be attached to a patient's finger and the blood pressure measurement controller 120 may be attached on the patient's hand with an attachment bracelet 121 that wraps around the patient's wrist. However, it should be appreciated that due to the small size of the blood pressure measurement controller 120 that a wide variety of attachment configurations may be utilized. For example, the blood pressure measurement controller 120 may be placed on a patient's finger (e.g., the same finger as the finger cuff 104 or on one or more different fingers), hand, wrist, arm, or other places such that it is mounted or placed locally to the finger cuff 104 in a convenient fashion. As one particular example, the blood pressure measurement controller 120 may be clipped to a pair of the patient other fingers (e.g., utilizing the attachment bracelet or simply a Velcro-strip). The attachment bracelet 121 may be metal, plastic, Velcro, etc.

Alternatively, the blood pressure measurement controller 120 may be placed not on the patient's body but may be placed or mounted in close proximity to the finger cuff 104. For example, the blood pressure measurement controller 120 may be clamped or attached to the arm rest 112 (e.g., placed on a clip or secured with Velcro) near the finger cuff 104 or may simply dangle off of the finger cuff 104 and may not be attached to anything. By having the blood pressure measurement controller 120 removed from the patient's body, access to a patient's arteries and veins is freed-up. Additionally, it should be appreciated that the approximately rectangular formation of the blood pressure measurement controller 120 shown in Figure 1 is merely a design implementation and that any suitable shape may be used. It should further be appreciated that due to the small size of the blood pressure measurement controller 120 that a wide variety of attachment configurations may be utilized, and these are merely examples.

It should be appreciated that a finger cuff connector 122 in accordance with embodiments of the invention may be utilized to connect a finger cuff 104 to either a blood pressure measurement controller 120 described herein, or a pressure generating and regulating system of any other kind, such as a conventional pressure generating and regulating system that is located remotely from the body of the patient (e.g., a pump 134 located remotely from a patient). Any kind of pressure generating and regulating system that can be used, including but not limited to the blood pressure measurement controller 120, may be described simply as a pressure generating and regulating system. As a further example, in some embodiments, there may be no blood pressure measurement controller 120, at all, and a remote pump 134 that is controlled remotely may be directly connected via a tube 136 and 123 to finger cuff connector 122 and finger cuff 104 to provide pneumatic pressure to the finger cuff 104.

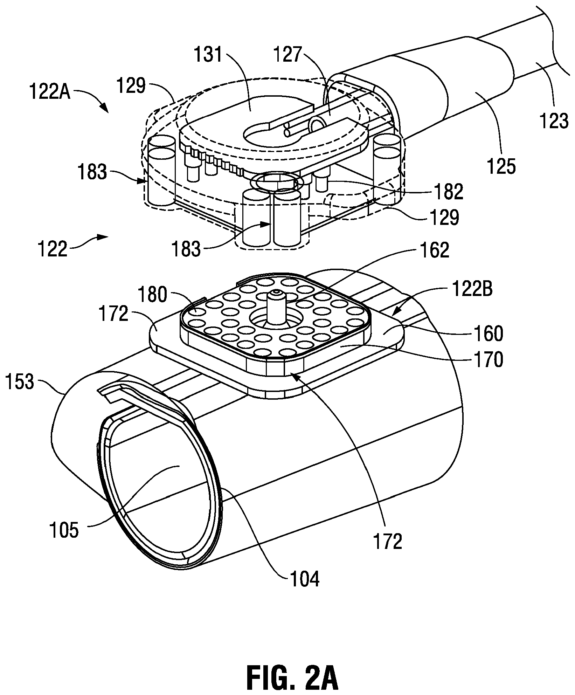

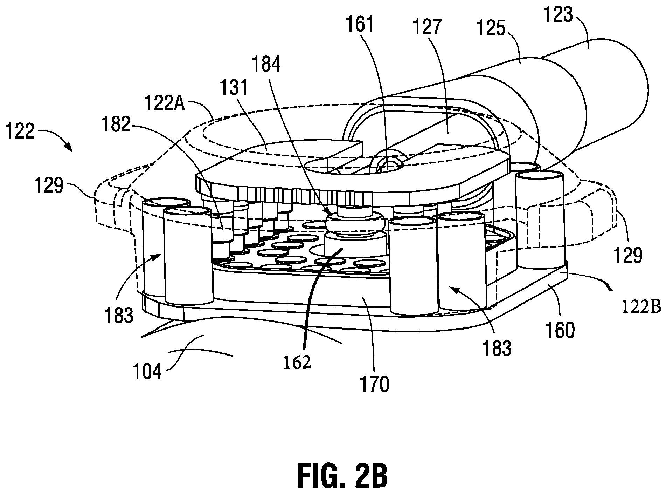

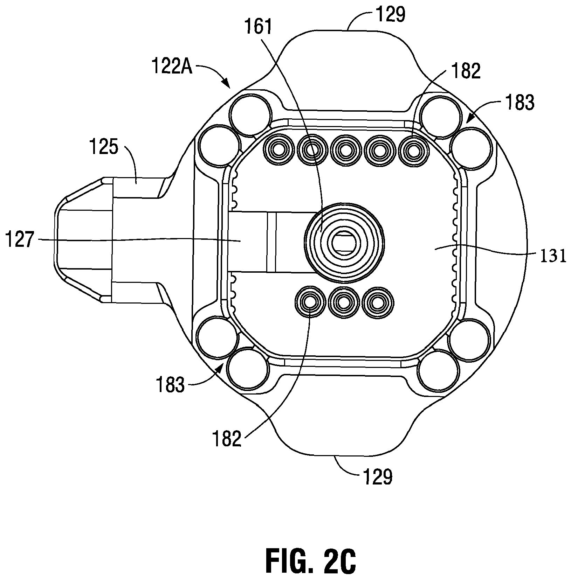

Referring to FIGs. 2A-2C, diagrams illustrating an embodiment of a finger cuff connector 122 according to the invention is shown. As has been described, the finger cuff connector 122 may be coupled to the finger cuff 104. The finger cuff 104 may include a suitable flexible circular structure to wrap the bladder 105 around a patient's finger and align the LED-PD pair (not shown) about the patient's finger and may have an extended clamping section 153 (e.g., Velcro on the interior) to clamp to the outside section (e.g., Velcro on the outside section) of the finger cuff 104 to firmly attach the finger cuff 104 to the patient's finger. The finger cuff connector 122 may be attached to the top portion of the finger cuff 104 as shown in FIGs. 2A and 2B.

The finger cuff connector 122 may include a cable portion 123, a connection portion 125, and a finger cuff connector pair (122A and 122B). The top finger cuff connector 122A housing may be approximately circular shape with two opposed protrusions 129 for ease of handling, placement, attachment, and rotation by a user to the bottom finger cuff connector 122B and the bottom finger cuff connector 122B may be approximately square shaped and the top and bottom finger cuff connectors 122A and 122B mate together, as will be described. The connection portion 125 connects the finger cuff connector pair 122A and 122B to the cable portion 123. The cable portion 123 may include a tube section for pneumatic pressure, as previously described, and, in particular, may include an appropriate pneumatic tube section 127 to provide pneumatic pressure to the bladder 105 of the finger cuff 104 and a suitable electrical connection (e.g., electrical wiring - not shown) to transmit the pleth signal received from the LED-PD pair of the finger cuff 104 to an appropriate computing device.

As previously described, the finger cuff connector pair 122 may comprise two halves: a first half 122A that is connected to the pressure generating and regulating system via the pneumatic tube section 127 (for the transmission of pneumatic pressure) and electrical wires (for transmitting and receiving electrical signals) of the cable portion 123; and a second half 122B that is fixedly attached to the finger cuff 104 on a square-shaped mounting plate 160. As can be seen in FIGs. 2A-2C, the top first half of finger cuff connector 122A has an open interior and an approximately square-shaped bottom section that contacts the outside sections of mounting plate 160 where they are connected. Further, as will be described, the interior portion of the top first half of finger cuff connector 122A surrounds an approximately square-shaped mounting section 170 in a manner that provides for a wide variety of possible orientations, as will be described in more detail hereafter. Also, the first half 122A may include a U-shaped printed circuit board portion 131 for mounting electrical connector pins 182 and connecting to the electrical wiring in the cable portion 123.

When the first top half 122A and second bottom half 122B are properly connected, electrical and pneumatic connections are arranged within each half of the finger cuff connector pair 122 such that when the first half 122A and the second half 122B of the finger cuff connector pair 122 are properly connected, suitable electrical and pneumatic connections are established between the pressure generating and regulating system and the finger cuff 104.

As an example, properly established electrical connections between the pressure generating and regulating system and the finger cuff 104 may include suitable power, data, and control signal connections between the circuitry of the pressure generating and regulating system and the circuitry of the finger cuff 104 (e.g., the LED-PD pair).

In one embodiment, to achieve suitable electrical connections, the second half 122B of the finger cuff connector pair 122 may comprise a plurality of electrical connector pads 180 that are located within the mounting section 170, in which the mounting section 170 includes an appropriate printed circuit board portion for the electrical connector pads 180. According to the invention the number of sets of electrical connector pads 180 is commensurate with the number of possible connector orientations such that there is a set of electrical connector pads 180 in the second half 122B that makes suitable contact with electrical connector pins 182 in the first half 122A when the two halves are connected in any of the possible orientations, as will be described. Of course, other types of electrical connections than pad-pin connections may also be utilized without deviating from the scope of the disclosure. When properly connected, in a particular orientation, data from the LED-PD pair of the finger cuff 104 may be transmitted through connector pads 180 and connector pins 182 through wires of cable portion 123 to the pressure generating and regulatory system for processing.

Further, properly established pneumatic connections between the pressure generating and regulating system and the finger cuff 104 enable a pump of the pressure generating and regulating system to provide pneumatic pressure to the bladder 105 of the finger cuff 104.

In one embodiment, pneumatic pressure from the pneumatic tube section 127 of cable portion 123 from the first half 122A may be connected to a tube 162 of the second half 122B, which is connected to the bladder 105 of the finger cuff 104. This connection may be made by an L-shaped connector tube 161 that is rotatably coupled to tube 162 by a suitable rotatable mounting device 184 (e.g., a rotatable seal). In this way, pneumatic pressure may be provided to the bladder 105 of the finger cuff 104 by the pressure generating system through the finger connector pair 122 when the two halves 122A and 122B are connected in any of the possible orientations, to be hereafter described.

As will be described, in different embodiments, the first half 122A and the second half 122B of the finger cuff connector pair 122 may be connected in a variety of different orientations (e.g., two or more possible orientations). For example, the first half 122A and the second half 122B may be connected in two orientations (e.g., 90 degrees), four orientations (e.g., 45 degrees), six orientations (60 degrees), or any number of different orientations. Also, in one embodiment, once the first half 122A and the second half 122B are connected or mated together, the first half 122A may rotate relative to the fixed second half 122B continuously within a plane.

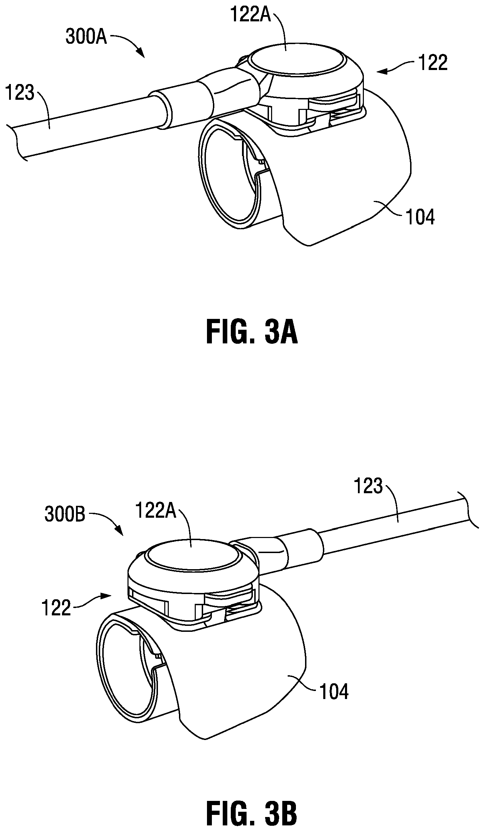

With additional reference to FIGs. 3A and 3B, diagrams 300A, 300B illustrate embodiments of finger cuff connector pairs 122 connected in two different orientations (e.g., 90 degrees). FIG. 3A shows the first half 122A and the second half (contained therein) of a finger cuff connector pair 122 connected in a rearward orientation. FIG. 3B shows the first half 122A and the second half (contained therein) of the finger cuff connector pair 122 connected in a forward orientation.

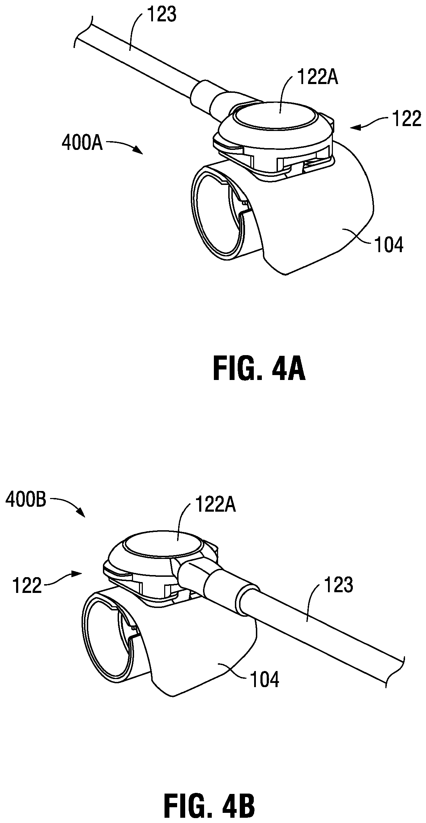

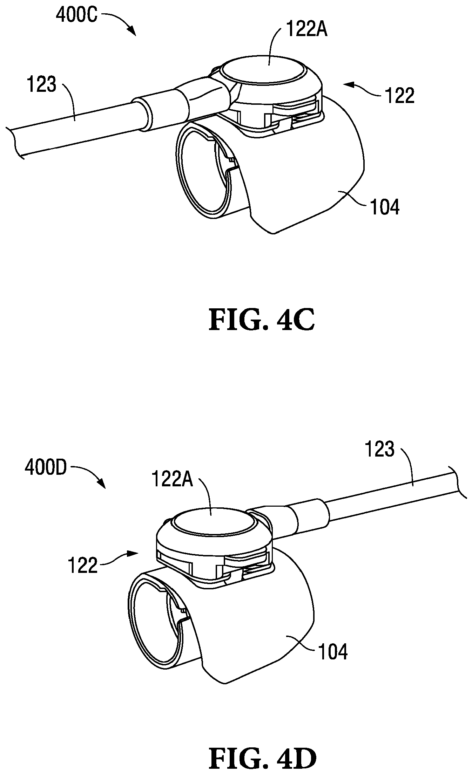

With additional reference to FIGs. 4A, 4B, 4C, and 4D, diagrams 400A, 400B, 400C, 400D illustrate embodiments of finger cuff connector pairs 122 connected in four different orientations (e.g., 45 degrees). FIG. 4A shows the first half 122A and the second half (contained therein) of a finger cuff connector pair 122 connected in a left orientation. FIG. 4B shows the first half 122A and the second half (contained therein) of the finger cuff connector pair 122 connected in a right orientation. FIG. 4C shows the first half 122A and the second half (contained therein) of the finger cuff connector pair 122 connected in a rearward orientation. FIG. 4D shows the first half 122A and the second half (contained therein) of the finger cuff connector pair 122 connected in a forward orientation.

With additional reference again to FIGs. 2A-2C, various implementations to achieve the different orientations of FIGs 3-4, as well as others, will be described.

In one embodiment, the first top half 122A and the second bottom half 122B of the finger cuff connector pair 122 may be connected in two or more discrete possible orientations, such as those shown in FIGs 3-4. In order to achieve this and according to the invention, mechanical key and magnetic features are utilized. In particular, the first half 122A of the finger cuff connector pair 122 may be rotated and positioned relative to the fixed second half 122B of the finger cuff connector pair 122 so as to facilitate proper alignment of the connector halves and may be attached to the fixed second half 122B in order to establish suitable electrical and pneumatic connections with the pressure generation and regulatory system.

In one embodiment, a keying feature in combination with a magnetic feature may be implemented to achieve four possible orientations. In this embodiment, the first top half 122A and the second bottom half 122B of the finger cuff connector pair 122 are connected or mated together. The top first half of finger cuff connector 122A has an open interior and an approximately square-shaped bottom section that contacts the outside sections of mounting plate 160, in which the mounting plate 160 may be formed of a magnetic material. Further, the top first half of finger cuff connector 122A includes four pairs of approximately cylindrically shaped magnets 183 that are located approximately at corners of the top first half of finger cuff connector 122A. The second bottom half of finger cuff connector 122B has an approximately square-shaped mounting section 170 that includes angled corners 172.

Therefore, as an example, a user may align the first top half 122A with the second bottom half 122B, in one of the four previously described orientations (e.g., FIGs. 3-4), to connect them together. In this connection operation, the interior portion of the top half 122A of the finger cuff connector surrounds the mounting section 170 of the fixed bottom half 122B so that the cylindrically shaped magnets 183 at the corners of the top half 122A mate with and abut the angled corners 172 of the bottom half 122B to properly align and connect in one of the four different orientation positions. In this way a keying feature is provided. Further, the cylindrically shaped magnets 183 abut against the mounting plate 160 and magnetically connect to the magnetic material of the mounting plate 160 such that the first and second halves are magnetically attached to one another (e.g., providing a more secure connection). It should be appreciated that this is just one example, and that a wide variety of orientations may possible, such as: two (90 degrees), six (60 degrees), eight (45 degrees) etc.; dependent upon design considerations.

As has been described, once the first top half 122A and the second bottom half 122B are connected together, suitable electrical connections may be achieved, by the electrical connector pins 182 of the first half 122A contacting the electrical connector pads 180 of the mounting section 170 of the second half 122B of the finger cuff. According to the invention the number of sets of electrical connector pads 180 is commensurate with the number of possible connector orientations such that there is a set of electrical connector pads 180 in the second half 122B that makes suitable contact with the electrical connector pins 182 in the first half 122A when the two halves are connected in any of the possible orientations. As can be seen in FIGs 2A-2C, sufficient electrical connector pads 180 are provided for connecting with the electrical connector pins 182 to provide for electrical connections for the two or four different orientations (e.g., FIGs. 3-4). In this way, electrical connections may be properly established between the pressure generating and regulating system and the finger cuff 104 and these electrical connections may include suitable power, data, and control signal connections between the circuitry of the pressure generating and regulating system and the circuitry of the finger cuff 104 (e.g., the LED-PD pair).

Further, when the first top half 122A is mated to the second bottom half 122B, pneumatic pressure from the pneumatic tube section 127 of the cable portion 123 from the first half 122A may be connected to the tube 162 of the second half 122B that is connected to the bladder 105 by the L-shaped connector tube 161 that is rotatably coupled to tube 162 by a suitable rotatable mounting device 184 (e.g., a rotatable seal). In this way, pneumatic pressure may be provided to the bladder 105 of the finger cuff 104 by the pressure generating system through the finger cuff connector 122 when the two halves 122A and 122B are connected in any of the possible orientations. In particular, pneumatic pressure may be provide to the bladder 105 of the finger cuff 104 in any of previously described orientations (e.g., the two or four different orientations (e.g., FIGs. 3-4)).

Also, various other different types of electrical connection methods may be utilized. For example, in one embodiment, switching circuitry may be utilized to reconfigure the electrical connectors in the first half 122A and/or the second half 122B based on the orientation in which the two halves are connected to ensure proper electrical connections.

Further, in another example of a finger cuff connector 122, the first half 122A of finger cuff connector 122 may rotate relative to the second half 122B of finger cuff connector in a plane when connected. In this example, the electrical connector pads of the second half 122B may be shaped in concentric rings to accommodate electrical connections with the electrical connector pins of the first half 122A.

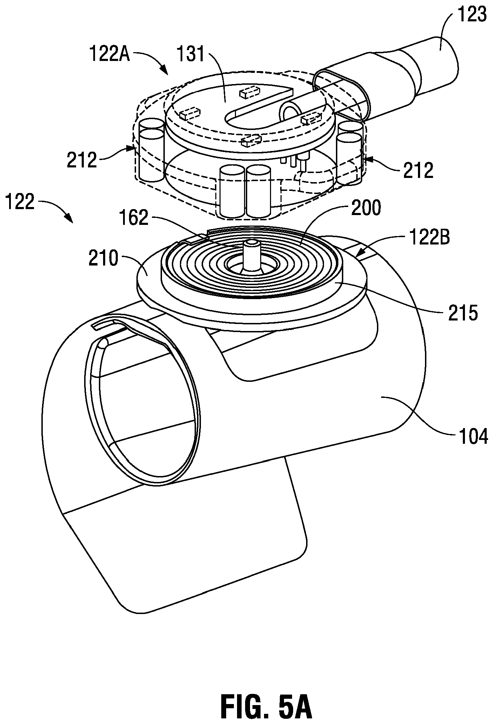

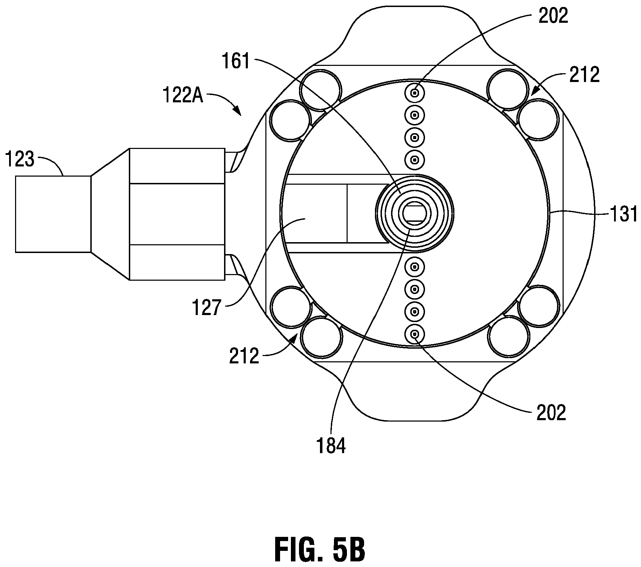

With additional reference to FIGs. 5A-5B, a further example of a finger cuff connector 122 in which the first half 122A of finger cuff connector 122 may rotate relative to the second half 122B of the finger cuff connector in a plane when connected, will be described. In the disclosed example, the electrical connector pads of the second half 122B may be shaped in concentric rings 200 to accommodate electrical connections with the electrical connector pins 202 of the first half 122A.

In this example, the first half 122A of finger cuff connector 122 may rotate relative to the second half 122B of finger cuff connector in a plane when connected such that any orientation position may be selectable by a user. In particular, the first half 122A of the finger cuff connector pair 122 may be positioned relative to the fixed second half 122B of the finger cuff connector pair 122 so as to facilitate proper alignment of the connector halves in any orientation position and may be attached to the fixed second half 122B in order to establish suitable electrical and pneumatic connections with the pressure generation and regulatory system.

Similar to the previously described example, the top first half of finger cuff connector 122A has an approximately circular open interior and an approximately square-shaped bottom section that contacts the outside sections of mounting plate 210, in which the mounting plate 210 may be formed of a magnetic material and is approximately circular shaped. Further, the top first half of finger cuff connector 122A includes four pairs of approximately cylindrically shaped magnets 212 that are located approximately at corners of the top first half of finger cuff connector 122A. The second bottom half of finger cuff connector 122B has an approximately circular-shaped mounting section 215. As an example, a user may align the first top half 122A with the second bottom half 122B to connect them together in any orientation position. In this connection operation, the interior portion of the top half 122A of the finger cuff connector surrounds the mounting section 215 of the fixed bottom half 122B and the cylindrically shaped magnets 212 at the corners of the top half 122A abut against both the circular mounting section 215 and the mounting plate 210 and magnetically connect to the magnetic material of the mounting plate 210 such that the first and second halves are magnetically attached to one another (e.g., providing a more secure connection). Further, this allows for the connection of the first and second halves 122A and 122B of the finger cuff connector in any orientation selected by the user.

Once the first top half 122A and the second bottom half 122B are connected together, suitable electrical connections may be achieved by the electrical connector pins 202 of the U-shaped printed circuit board portion 131 (that connect to electrical wiring in the cable portion 123) contacting the electrical concentric connector pad rings 200 of the mounting section 215 of the second half 122B of the finger cuff 104. In this way, electrical connections may be properly established between the pressure generating and regulating system and the finger cuff 104 and these electrical connections may include suitable power, data, and control signal connections between the circuitry of the pressure generating and regulating system and the circuitry of the finger cuff 104 (e.g., the LED-PD pair). Further, when the first top half 122A is mated to the second bottom half 122B, pneumatic pressure from the pneumatic tube section 127 of the cable portion 123 from the first half 122A may be connected to the tube 162 of the second half 122B that is connected to the bladder by the L-shaped connector tube 161, as has been previously described. The pneumatic connections occur in the same way as the previously described embodiments, such that pneumatic pressure may be provided to the bladder of the finger cuff 104 by the pressure generating system through the finger cuff connector 122 when the two halves 122A and 122B are connected in any of the possible rotatable orientations. Therefore, in this example, the first half 122A of finger cuff connector 122 may rotate relative to the second half 122B of finger cuff connector in a plane when connected such that any orientation position may be selectable by a user.

It should be appreciated that various different types of mechanisms may be utilized in addition to or instead of the previously described mechanisms to retain the physical connection between the first half 122A and the second half 122B of the finger cuff connector 122. For example, these mechanisms may include: other types of magnetic retention mechanisms, a snap mechanism, a twist-on mechanism, a press-fit mechanism, a cam latch, or any other suitable mechanism. Thus, various other mechanisms, such as suitable mechanical, magnetic, or electro-mechanical mechanisms may also be utilized to facilitate various different types of orientations and proper alignment.

It should be appreciated that the physical interaction between the finger cuff and the patient's finger is critical for achieving a properly fitted and calibrated system such that the pressure in the finger cuff is equal to the pressure in the patient's artery (e.g., such that the transmural pressure drop is negligible). Mechanical forces exerted on the finger cuff by the cable can affect the fit and interaction between the finger cuff and the patient's finger and thereby interfere with accurate, continuous blood pressure measurement.

By utilizing the multiple types of connector orientations, according to the embodiments of the invention previously described, a great degree of flexibility is provided to configure the cable in a manner that reduces the forces exerted on the patient's finger. This type of flexibility is greatly needed to accommodate variations in patient position and the positions of supporting equipment during surgery and in the intensive care unit (ICU), emergency room (ER), and other locations.

It should be appreciated that aspects of the invention previously described may be implemented in conjunction with the execution of instructions by processors, circuitry, controllers, control circuitry, etc. As an example, control circuity may operate under the control of a program, algorithm, routine, or the execution of instructions to execute methods or processes in accordance with embodiments of the invention previously described. For example, such a program may be implemented in firmware or software (e.g. stored in memory and/or other locations) and may be implemented by processors, control circuitry, and/or other circuitry, these terms being utilized interchangeably. Further, it should be appreciated that the terms processor, microprocessor, circuitry, control circuitry, circuit board, controller, microcontroller, etc., refer to any type of logic or circuitry capable of executing logic, commands, instructions, software, firmware, functionality, etc., which may be utilized to execute embodiments of the invention.

The various illustrative logical blocks, processors, modules, and circuitry described in connection with the embodiments disclosed herein may be implemented or performed with a general purpose processor, a specialized processor, circuitry, a microcontroller, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A processor may be a microprocessor or any conventional processor, controller, microcontroller, circuitry, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module/firmware executed by a processor, or any combination thereof. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such that the processor can read information from, and write information to, the storage medium. In the alternative, the storage medium may be integral to the processor.

## Claims

1. A connector (122) for a blood pressure measurement system (102) that includes a pressure generating and regulating system and a finger cuff (104), the connector (122) comprising:
a first half portion (122A) pneumatically and electrically connected to the pressure generating and regulating system; and
a second half portion (122B) fixedly attached to the finger cuff (104), wherein the first half portion (122A) and the second half portion (122B) are connectable in two or more orientations, and wherein the pressure generating and regulating system and the finger cuff (104) are pneumatically and electrically connected when the first half portion (122A) and the second half portion (122B) are connected,
wherein the first half portion (122A) and the second half portion (122B) comprise one or more mechanical key features that facilitate alignment of the first half portion (122A) and the second half portion (122B)
**characterized in that** the mechanical key features facilitate alignment of the first half portion (122A) and the second half portion (122B) in two or more discrete possible angular orientations, wherein the connector (122) comprises electrical connector pads (180) being commensurate with the number of possible orientations such that there is a set of electrical connector pads (180) in the second half portion (122B) that makes suitable contact with electrical connector pins (182) in the first half portion (122A) when the two halves are connected in any of the possible orientations.

2. The connector (122) of claim 1, wherein the first half portion (122A) and the second half portion (122B) are connectable in two, four, or six orientations.

3. The connector (122) of claim 1, wherein the second half portion (122B) comprises two or more discrete sets of electrical connection pads (180).

4. The connector (122) of claim 3, wherein each set of electrical connection pads (180) corresponds to one orientation in which the first half portion (122A) and the second half portion (122B) are connectable.

5. The connector (122) of claim 1, further comprising a magnetic retention mechanism.

6. The connector (122) of claim 1, further comprising one or more of: a snap mechanism, a twist-on mechanism, a press-fit mechanism, or a cam latch.

7. The connector (122) of claim 1, wherein the pressure generating and regulating system provides pneumatic pressure to an inflatable bladder of the finger cuff (104) when the first half portion (122A) and the second half portion (122B) are connected.

8. A method for applying a connector (122) to a finger cuff (104) of a blood pressure measurement system that includes a pressure generating and regulating system for measuring a patient's blood pressure, the method comprising:
attaching the finger cuff (104) to the patient's finger; and
connecting a first half portion (122A) of the connector to a second half portion (122B) of the connector (122), the second half portion (122B) of the connector being fixedly attached to the finger cuff (104) and the first half portion (122A) being pneumatically and electrically connected to the pressure generating and regulating system, wherein the first half portion (122A) and the second half portion (122B) are connectable in two or more orientations, and wherein the pressure generating and regulating system and the finger cuff (104) are pneumatically and electrically connected when the first half portion (122A) and the second half portion (122B) are connected,
wherein the first half portion (122A) and the second half portion (122B) comprise one or more mechanical key features that facilitate alignment of the first half portion (122A) and the second half portion (122B)
**characterized in that** the mechanical key features facilitate alignment of the first half portion (122A) and the second half portion (122B) in two or more discrete possible angular orientations, wherein the connector (122) comprises electrical connector pads (180) being commensurate with the number of possible orientations such that there is a set of electrical connector pads (180) in the second half portion (122B) that makes suitable contact with electrical connector pins (182) in the first half portion (122A) when the two halves are connected in any of the possible orientations.

9. The method of claim 8, wherein a magnetic retention mechanism is utilized in connecting the first half portion (122A) and the second half portion (122B) of the connector.

10. The method of claim 8, further comprising one or more of: a snap mechanism, a twist-on mechanism, a press-fit mechanism, or a cam latch; in connecting the first half portion (122A) and the second half portion (122B) of the connector.

11. A blood pressure measurement system that includes a pressure generating and regulating system to measure a patient's blood pressure, the blood pressure measurement system comprising:
a finger cuff (104) attached to the patient's finger; and
a connector (122) according to any one of claims 1 to 7.

## Patentansprüche

1. Verbinder (122) für ein Blutdruckmesssystem (102), das ein Druckerzeugungs- und Druckregulierungssystem und eine Fingermanschette (104) aufweist, wobei der Verbinder (122) umfasst:
einen ersten Halbabschnitt (122A), der pneumatisch und elektrisch mit dem Druckerzeugungs- und Druckregulierungssystem verbunden ist; und
einen zweiten Halbabschnitt (122B), der fest an der Fingermanschette (104) angebracht ist, wobei der erste Halbabschnitt (122A) und der zweite Halbabschnitt (122B) in zwei oder mehr Ausrichtungen verbindbar sind, und wobei das Druckerzeugungs- und Druckregulierungssystem und die Fingermanschette (104) pneumatisch und elektrisch verbunden sind, wenn der erste Halbabschnitt (122A) und der zweite Halbabschnitt (122B) verbunden sind,
wobei der erste Halbabschnitt (122A) und der zweite Halbabschnitt (122B) einen oder mehrere mechanische Schlüsselmerkmale umfassen, die das Ausrichten des ersten Halbabschnitts (122A) und des zweiten Halbabschnitts (122B) erleichtern,
**dadurch gekennzeichnet, dass** die mechanischen Schlüsselmerkmale das Ausrichten des ersten Halbabschnitts (122A) und des zweiten Halbabschnitts (122B) in zwei oder mehr diskreten möglichen Winkelausrichtungen erleichtern, wobei der Verbinder (122) elektrische Verbinderflächen (180) umfasst, die der Anzahl der möglichen Ausrichtungen entsprechen, so dass eine Menge von elektrischen Verbinderflächen (180) in dem zweiten Halbabschnitt (122B) vorhanden ist, die einen geeigneten Kontakt mit elektrischen Verbinderstiften (182) in dem ersten Halbabschnitt (122A) herstellen, wenn die beiden Hälften in einer der möglichen Ausrichtungen verbunden sind.

2. Verbinder (122) nach Anspruch 1, wobei der erste Halbabschnitt (122A) und der zweite Halbabschnitt (122B) in zwei, vier oder sechs Ausrichtungen verbindbar sind.

3. Verbinder (122) nach Anspruch 1, wobei der zweite Halbabschnitt (122B) zwei oder mehr diskrete Mengen von elektrischen Verbinderflächen (180) aufweist.

4. Verbinder (122) nach Anspruch 3, wobei jede Menge der elektrischen Verbinderflächen (180) einer Ausrichtung entspricht, in welcher der erste Halbabschnitt (122A) und der zweite Halbabschnitt (122B) verbindbar sind.

5. Verbinder (122) nach Anspruch 1, weiter einen magnetischen Haltemechanismus umfassend.

6. Verbinder (122) nach Anspruch 1, weiter umfassend einen oder mehrere von: einem Schnappmechanismus, einem Drehmechanismus, einem Einpressmechanismus oder einer Nockenverriegelung.

7. Verbinder (122) nach Anspruch 1, wobei das Druckerzeugungs- und Druckregulierungssystem einen pneumatischen Druck für eine aufpumpare Blase der Fingermanschette (104) bereitstellt, wenn der erste Halbabschnitt (122A) und der zweite Halbabschnitt (122B) verbunden sind.

8. Verfahren zur Anwendung eines Verbinders (122) an einer Fingermanschette (104) eines Blutdruckmesssystems, das ein Druckerzeugungs- und Druckregulierungssystem zur Messung eines Blutdrucks des Patienten aufweist, wobei das Verfahren umfasst:
Anbringen der Fingermanschette (104) an dem Patientenfinger; und
Verbinden eines ersten Halbabschnitts (122A) des Verbinders mit einem zweiten Halbabschnitt (122B) des Verbinders (122), wobei der zweite Halbabschnitt (122B) des Verbinders fest an der Fingermanschette (104) angebracht ist und der erste Halbabschnitt (122A) pneumatisch und elektrisch mit dem Druckerzeugungs- und Druckregulierungssystem verbunden ist, wobei der erste Halbabschnitt (122A) und der zweite Halbabschnitt (122B) in zwei oder mehr Ausrichtungen verbindbar sind, und wobei das Druckerzeugungs- und Druckregulierungssystem und die Fingermanschette (104) pneumatisch und elektrisch verbunden sind, wenn der erste Halbabschnitt (122A) und der zweite Halbabschnitt (122B) verbunden sind,
wobei der erste Halbabschnitt (122A) und der zweite Halbabschnitt (122B) ein oder mehr mechanische Schlüsselmerkmale umfassen, die das Ausrichten des ersten Halbabschnitts (122A) und des zweiten Halbabschnitts (122B) erleichtern,
**gekennzeichnet dadurch, dass** die mechanischen Schlüsselmerkmale die Ausrichtung des ersten Halbabschnitts (122A) und des zweiten Halbabschnitts (122B) in zwei oder mehr diskreten möglichen Winkelausrichtungen erleichtern, wobei der Verbinder (122) elektrische Verbinderflächen (180) umfasst, die der Anzahl der möglichen Ausrichtungen entsprechen, so dass eine Menge von elektrischen Verbinderflächen (180) in dem zweiten Halbabschnitt (122B) vorhanden ist, die einen geeigneten Kontakt mit elektrischen Verbinderstiften (182) in dem ersten Halbabschnitt (122A) herstellen, wenn die beiden Hälften in einer der möglichen Ausrichtungen verbunden sind.

9. Verfahren nach Anspruch 8, wobei ein magnetischer Haltemechanismus verwendet wird, um den ersten Halbabschnitt (122A) und den zweiten Halbabschnitt (122B) des Verbinders zu verbinden.

10. Verfahren nach Anspruch 8, beim Verbinden des ersten Halbabschnitts (122A) und des zweiten Halbabschnitts (122B) des Verbinders weiter umfassend einen oder mehrere von: einem Schnappmechanismus, einem Drehmechanismus, einem Einpressmechanismus oder einer Nockenverriegelung.

11. Blutdruckmesssystem, das ein Druckerzeugungs- und Druckregulierungssystem aufweist, um den Blutdruck eines Patienten zu messen, wobei das Blutdruckmesssystem umfasst:
eine Fingermanschette (104), die an dem Patientenfinger angebracht ist; und
einen Verbinder (122) nach einem der Ansprüche 1 bis 7.

## Revendications

1. Raccord (122) pour un système de mesure de tension artérielle (102) qui comprend un système de génération et de régulation de pression et un doigtier (104), le raccord (122) comprenant :
une première moitié (122A) raccordée pneumatiquement et électriquement au système de génération et de régulation de pression ; et
une seconde moitié (122B) fixée de manière fixe au doigtier (104), la première moitié (122A) et la seconde moitié (122B) pouvant être raccordées dans deux orientations ou plus et le système de génération et de régulation de pression et le doigtier (104) étant raccordés pneumatiquement et électriquement lorsque la première moitié (122A) et la seconde moitié (122B) sont raccordées,
la première moitié (122A) et la seconde moitié (122B) comprenant une ou plusieurs caractéristiques clés mécaniques qui facilitent l'alignement de la première moitié (122A) et de la seconde moitié (122B)
**caractérisé en ce que** les caractéristiques clés mécaniques facilitent l'alignement de la première moitié (122A) et de la seconde moitié (122B) dans deux orientations angulaires possibles discrètes ou plus, le raccord (122) comprenant des plots de connecteur électrique (180) correspondant au nombre d'orientations possibles de telle sorte qu'il existe un ensemble de plots de connecteur électrique (180) dans la seconde moitié (122B) qui réalise un contact approprié avec des broches de connecteur électrique (182) dans la première moitié (122A) lorsque les deux moitiés sont raccordées dans l'une quelconque des orientations possible.

2. Raccord (122) selon la revendication 1, la première moitié (122A) et la seconde moitié (122B) pouvant être raccordées dans deux, quatre ou six orientations.

3. Raccord (122) selon la revendication 1, la seconde moitié (122B) comprenant deux ensembles discrets ou plus de plots de connexion électrique (180).

4. Raccord (122) selon la revendication 3, chaque ensemble de plots de connexion électrique (180) correspondant à une orientation dans laquelle la première moitié (122A) et la seconde moitié (122B) peuvent être raccordées.

5. Raccord (122) selon la revendication 1, comprenant en outre un mécanisme de rétention magnétique.

6. Raccord (122) selon la revendication 1, comprenant en outre un ou plusieurs éléments parmi : un mécanisme d'encliquetage, un mécanisme de verrouillage par rotation, un mécanisme à ajustage serré ou un verrou à came.

7. Raccord (122) selon la revendication 1, le système de génération et de régulation de pression fournissant une pression pneumatique à une poche gonflable du doigtier (104) lorsque la première moitié (122A) et la seconde moitié (122B) sont raccordées.

8. Procédé d'application d'un raccord (122) à un doigtier (104) d'un système de mesure de tension artérielle qui comprend un système de génération et de régulation de pression pour mesurer la tension artérielle d'un patient, le procédé comprenant :
la fixation du doigtier (104) au doigt du patient ; et
le raccordement d'une première moitié (122A) du raccord à une seconde moitié (122B) du raccord (122), la seconde moitié (122B) du raccord étant fixée de manière fixe au doigtier (104) et la première moitié (122A) étant raccordée pneumatiquement et électriquement au système de génération et de régulation de pression, la première moitié (122A) et la seconde moitié (122B) pouvant être raccordées dans deux orientations ou plus et le système de génération et de régulation de pression et le doigtier (104) étant raccordés pneumatiquement et électriquement lorsque la première moitié (122A) et la seconde moitié (122B) sont raccordées,
la première moitié (122A) et la seconde moitié (122B) comprenant une ou plusieurs caractéristiques clés mécaniques qui facilitent l'alignement de la première moitié (122A) et de la seconde moitié (122B)
**caractérisé en ce que** les caractéristiques clés mécaniques facilitent l'alignement de la première moitié (122A) et de la seconde moitié (122B) dans deux orientations angulaires possibles discrètes ou plus, le raccord (122) comprenant des plots de connecteur électrique (180) correspondant au nombre d'orientations possibles de telle sorte qu'il existe un ensemble de plots de connecteur électrique (180) dans la seconde moitié (122B) qui réalise un contact approprié avec des broches de connecteur électrique (182) dans la première moitié (122A) lorsque les deux moitiés sont raccordées dans l'une quelconque des orientations possibles.

9. Procédé selon la revendication 8, un mécanisme de rétention magnétique étant utilisé pour raccorder la première moitié (122A) et la seconde moitié (122B) du raccord.

10. Procédé selon la revendication 8, comprenant en outre un ou plusieurs éléments parmi : un mécanisme d'encliquetage, un mécanisme de verrouillage par rotation, un mécanisme à ajustage serré ou un verrou à came dans le raccordement de la première moitié (122A) et de la seconde moitié (122B) du raccord.

11. Système de mesure de tension artérielle qui comprend un système de génération et de régulation de pression pour mesurer la tension artérielle d'un patient, le système de mesure de tension artérielle comprenant :
un doigtier (104) fixé au doigt du patient ; et
un raccord (122) selon l'une quelconque des revendications 1 à 7.
